(19) Europäisches Patentamt
European Patent Office
Office européen des brevets

(11) **EP 4 570 163 B1**

(12) **EUROPEAN PATENT SPECIFICATION**

(45) Date of publication and mention
of the grant of the patent:
**29.07.2026 Bulletin 2026/31**

(21) Application number: **24219092.4**

(22) Date of filing: **11.12.2024**

(51) International Patent Classification (IPC):
***A61B 3/10*** *(2006.01)*     ***G01N 21/64*** *(2006.01)*

(52) Cooperative Patent Classification (CPC):
**A61B 3/10; A61B 5/0071; A61B 5/0075;
G01N 21/6408; G01N 21/6458**

(54) **SYSTEM AND METHOD FOR TWO-PHOTON FLUORESCENCE LIFETIME IMAGING OF FLUOROPHORES IN AN EYE**

SYSTEM UND VERFAHREN ZUR ZWEIPHOTONEN-FLUORESZENZLEBENSDAUERBILDGEBUNG VON FLUOROPHOREN IN EINEM AUGE

SYSTÈME ET MÉTHODE POUR L'IMAGERIE DE LA DURÉE DE VIE DE LA FLUORESCENCE À DEUX PHOTONS DE FLUOROPHORES DANS UN OEIL

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **12.12.2023 EP 23461687**

(43) Date of publication of application:
**18.06.2025 Bulletin 2025/25**

(73) Proprietor: **Instytut Chemii Fizycznej Polskiej Akademii Nauk**
**01-224 Warszawa (PL)**

(72) Inventors:
- **WOJTKOWSKI, Maciej**
  **01-224 Warszawa (PL)**
- **DABROWSKI, Micha**
  **01-224 Warszawa (PL)**

(74) Representative: **AOMB Polska Sp. z.o.o.**
**ul. Rondo Ignacego Daszynskiego 1**
**00-843 Warsaw (PL)**

(56) References cited:
**WO-A1-2022/204248**     **CN-A- 101 181 152**
**US-A1- 2020 378 830**

- **ANONYMOUS, SPIE, PO BOX 10 BELLINGHAM WA 98227-0010, USA, 31 December 2011 (2011-12-31), XP040561064**

**Description**

TECHNICAL FIELD

The present invention relates to in vivo eye measurements using two-photon excitation microscopy. More specifically, the present invention relates to systems and methods of two-photon fluorescence lifetime imaging of fluorophores in the eye.

BACKGROUND OF THE DISCLOSURE

Human eye contains a variety of endogenous fluorophores that play critical roles in physiological and pathological processes. The precise identification, quantification, and spatial distribution of these fluorophores are essential for understanding eye health, diagnosing diseases, and monitoring therapeutic interventions. Exogenous fluorophores, which facilitate targeted imaging and functional studies, may be introduced to the eye through dyes or markers. Accurate identification, quantification, and spatial mapping of endogenous or exogenous fluorophores are essential for diagnosing, monitoring, and understanding ocular diseases such as age-related macular degeneration (AMD), diabetic retinopathy, glaucoma, and other degenerative or metabolic disorders. Therefore, the knowledge about their presence and possibility of their qualitative and quantitative analysis are of diagnostic value as the presence of some of these fluorophores is a signature of abnormalities in the functionality of the visual cycle. Endogenous fluorophores are found in various ocular tissues, including the cornea, lens, retina, and retinal pigment epithelium (RPE). Key endogenous fluorophores are for example nicotinamide adenine dinucleotide (NADH), flavin adenine dinucleotide (FAD), lipofuscin, A2E (N-retinylidene-N-retinylethanolamine), melanin, collagen, elastin or aromatic amino acids.

Changes in the NADH/NAD$^+$ ratio indicate shifts in cellular metabolism and can be used to assess mitochondrial dysfunction. The FAD/NADH ratio may be used as a metabolic marker for oxidative stress. Lipofuscin accumulates with age and is a biomarker for age-related macular degeneration (AMD), Stargardt disease, and other retinal degenerative disorders. A2E contributes to oxidative stress and cell death in retinal diseases such as AMD. Changes in melanin distribution or concentration are associated with diseases such as melanoma. Alterations in collagen are used to study corneal diseases. Age-related changes in elastin contribute to diseases such as glaucoma. Protein denaturation and aggregation in the lens lead to cataract formation, which can be detected by changes in tryptophan fluorescence.

Various fluorescence-based imaging and spectroscopic techniques which enable analysis of fluorophores in the eye have been developed. These methods exploit the unique optical properties of fluorophores, including their emission spectra, fluorescence intensity, and fluorescence lifetime, to deliver information regarding ocular structures and functions. In a known state of the art, arrangement of the system could in principle be realized with a spectrometer or a monochromator as the final spectrum-measuring device. However, a significant challenge arises from the extremely low signal levels inherent to such measurements, often in the order of less than one photon per pixel. This presents a substantial obstacle to achieving reliable spectral data acquisition in a reasonable timeframe. This situation forces the use of a photomultiplier as a detector which eliminates the possibility of spectral measurements, since the signal acquisition would be too long to capture a meaningful set of data from one eye position. The prolonged exposure times necessary to accumulate sufficient photons would lead to issues such as motion artifacts, photobleaching of the fluorophores, and significant patient discomfort, particularly in a dynamic environment like the human eye. On the other hand, increasing the signal level would require an increase of the illuminating light intensity which is not possible due to safety limits. Excessive light intensity can cause photochemical damage to the retina or other ocular tissues, leading to temporary or even permanent vision impairment.

A one known method for fluorophore identification and analysis in ocular tissues is confocal laser scanning microscopy (CLSM), which is a high-resolution imaging technique that utilizes a laser to excite fluorophores and a pinhole to eliminate out-of-focus light, providing precise imaging of specific focal planes. This technique is frequently used in corneal imaging and in studies of retinal layers to visualize the spatial distribution of fluorophores. Although CLSM offers high spatial resolution and depth selectivity, it is limited to shallow tissue depths and can induce photobleaching and phototoxicity during prolonged imaging sessions.

Fundus autofluorescence (FAF) is a non-invasive imaging technique that detects the natural autofluorescence emitted by fluorophores such as lipofuscin within the retinal pigment epithelium and is commonly used to monitor the progression of retinal diseases. However, the autofluorescence signal can be weak and affected by lens opacities, and the technique has limited ability to differentiate between different fluorophores.

Fluorescence correlation spectroscopy (FCS) is a technique that analyses fluorescence intensity fluctuations over time to determine the diffusion coefficients and concentration of fluorophores. FCS is sensitive to single molecules and offers quantitative data on molecular diffusion and interactions. However, this technique has some limitations related to misleading calculations under different environments.

Another known method is two-photon excitation microscopy (TPEF), which is an advanced imaging method that uses two low-energy photons to excite a fluorophore, resulting in the emission of a single photon. This non-linear excitation process allows for deeper tissue penetration with reduced phototoxicity. TPEF minimizes photobleaching and phototoxicity, making it suitable for in vivo imaging. TPEF is particularly useful for imaging deep ocular structures, including the retina and optic nerve, to study cellular and metabolic processes. Nevertheless, the known method is limited by inaccuracies resulting from overlapping spectra of various fluorophores.

In general, known approaches are often limited by factors such as photobleaching, concentration dependence, and interference from overlapping signals. Often advanced deconvolution algorithms are required to separate overlapping spectra. In the area of two-photon fluorescence microscopic measurements, in the situations where it is desired to distinguish between a number of fluorophores emitting in a similar spectral bandwidth, a technique of fluorescence lifetime imaging microscopy (FLIM) may be used. FLIM is a non-invasive imaging technique that measures the fluorescence lifetime - the time a fluorophore remains in its excited state before emitting a photon. In this technique, temporal properties of the fluorescence decay are captured to identify the particular compounds, based on their physical properties, known from elsewhere. FLIM provides information about the molecular microenvironment, such as pH and oxygen levels, which influence the lifetime of fluorophores, and is capable of detecting subtle changes in the metabolic states of tissues. However, FLIM requires sophisticated instrumentation and the interpretation of fluorescence lifetime data can be complex due to the influence of various environmental factors. Therefore, as this method is complicated and lengthy, it is not suitable for the use in a setting of in-vivo human eye measurement.

An approach to achieve the separation of signals from different fluorophores is the phasor analysis technique. This is technically simpler to arrange and does not slow down the data acquisition significantly or unduly decrease the signal level. Phasor analysis is based on the representation of fluorescence decay signals in the frequency domain. In this method, the fluorescence decay of a fluorophore is transformed into a two-dimensional phasor plot by calculating the sine and cosine Fourier transforms of the fluorescence signal.

The resulting phasor coordinates are plotted on a Cartesian plane known as the phasor plot. Each fluorophore with a distinct fluorescence lifetime is represented as a unique point on the phasor plot, with the coordinates determined by its characteristic lifetime. Mixtures of fluorophores with different lifetimes appear as linear combinations of the individual phasor coordinates, allowing for the separation and quantification of multiple fluorophores in a single measurement.

From document WO2022/204248A1 a imaging system with the features of the preamble of claim 1 is known.

There is a need to address the disadvantages of the existing methods discussed above, particularly those related to their limitations or unsuitability for use in *in vivo* human eye measurements.

## SUMMARY OF THE INVENTION

The invention is defined in claim 1.

There is provided a two-photon fluorescence lifetime imaging system for imaging fluorophores in a living eye, comprising a laser for providing excitation light; a scanner for directing the laser light across the measured object; a detector for collecting the emitted light from the fluorophores; a dichroic mirror positioned in the optical path between the laser and the measured object, whereby the dichroic mirror reflects the excitation light and transmits the emission light from the measured object towards the detector. The system comprises a set of spectral filters positioned on the optical path between the dichroic mirror and the detector.

The set of spectral filters comprises a first filter having sine-wave spectral transmission characteristics, a second filter having cosine-wave spectral transmission characteristics, and a third filter being a bandpass filter. The transmission range of the third filter matches the emission spectrum of the fluorophore of interest.

The data obtained using the set of optical spectral filters is data suitable for performing phasor analysis. This further allows to perform spectroscopic analysis of the imaged retinal fluorophores without engaging fluorescence lifetime analysis or direct spectral measurements. Moreover, it allows for performing a spectroscopic analysis of the imaged retinal fluorophores in a short time and for relatively low signals.

The first filter and/or the second filter is for example selected from a group including: a Fabry-Pérot interferometer or a filter with dielectric multilayer coating or a thin-film filter or a holographic optical element.

Preferably, the free spectral range of the first filter and/or the second filter is 300 nm.

In the simplest example, the first filter, the second filter and the third filter are arranged separately.

Alternatively, the first filter, the second filter and the third filter may be mounted in a rotating disc. This facilitates the measurement process by allowing to quickly swap the filters during the procedure. The rotatable disc can be rotated to a desired position manually or electrically.

To further accelerate the measurement process, the rotatable disc is electrically operable.

The first filter, the second filter and the third filter are for example arranged concentrically around the center of the rotatable disc.

Alternatively, the first filter, the second filter and the third filter are arranged asymmetrically around the center of the rotatable disc or at uneven intervals around the center of the rotatable disc.

Preferably, the system further comprises a number of objective lenses for focusing the laser light and collecting fluorescence emission light.

Preferably, the laser emits light in the wavelength range between 700 nm to 1100 nm. This range covers most of the fluorophores, as wavelength range able to excite various fluorophores is typically in the range between 700 nm to 1100 nm.

Preferably, the third filter is a multiband bandpass filter, what allows to transmit fluorescence from multiple fluorophores simultaneously.

Preferably, the third filter has a center wavelength in the range from 400 to 700 nm.

Further, a method for two-photon fluorescence lifetime imaging of fluorophores in a living eye is provided. The method is performed in the system as described above, and comprises the following steps:

a) sending excitation light to an object to be measured;

b) reflecting the excitation light and transmitting emission light from the measured object towards an detector, whereby capturing the emission light from the measured object by the detector is performed in at least one frame series comprising sequences captured using a set of three spectral filters, each sequence having at least one frame.

Said frame series comprises capturing: (i) a sequence in which the emission light passes through a first filter having sine-wave spectral transmission characteristics; (ii) a sequence in which the emission light passes through a second filter having cosine-wave spectral transmission characteristics; and (iii) a sequence in which the emission light passes through a third filter being a bandpass filter. The transmission range of the third filter matches the emission spectrum of the fluorophore of interest. The steps i, ii, iii may be performed in any order.

The sequences described in steps i and iii may be performed simultaneously. Similarly, also the sequences described in ii. and iii. may be performed simultaneously.

The method may further comprise a step of performing spectral phasor analysis of the data obtained according to steps i, ii, iii..

The optional and/or preferred features of each aspect of the disclosure as set out herein are also applicable to any other aspects of the disclosure where appropriate.

BRIEF DESCRIPTION OF THE DRAWINGS

The disclosure of embodiments of the present invention will now be discussed, by way of example only, with reference to the accompanying drawings in which:

Figure 1 shows a scheme of the system for measuring the in-vivo fluorescence from the eye;
Figure 2 shows bandpass, sine- and cosine-shaped optical filter transmission characteristics which can be used to obtain dataset for phasor analysis;
Figure 3 shows an exemplary phasor representation showing a result for an exemplary fluorophore;
Figure 4 shows a scheme of the system for measuring the in-vivo fluorescence from the eye according to the present invention, where the set of spectral filters is mounted in a rotatable disc;
Figure 5A shows an exemplary embodiment of the rotatable disc comprising three spectral filters;
Fig. 5B shows another exemplary embodiment of the rotatable disc comprising three spectral filters.

DETAILED DESCRIPTION OF THE DISCLOSURE

In the context of measurements of the in-vivo two-photon fluorescence from the eye, it is desired to introduce a means of distinguishing the fluorophores - light emitting substances present in the eye. As mentioned above, the possibility of their qualitative and quantitative analysis is of diagnostic value as the presence of various fluorophores is a signature of ocular abnormalities or disorders. In the present method, this information is naturally overlaid on the image of the retina, and thus it brings the potential of a localized treatment.

According to one aspect of the present disclosure, a two-photon fluorescence measurement system is provided, dedicated to in-vivo measurement of an eye, in particular human eye. The system is capable of acquiring data prepared for spectral phasor analysis. a technique that enables the visualization and interpretation of complex fluorescence signals in a simplified, graphical form.

Further provided is a method of measuring two-photon fluorescence from the eye in vivo. In such method, a set of data is created that allows the identification and analysis of the quantity and position of fluorophores, without the need to perform FLIM measurements.

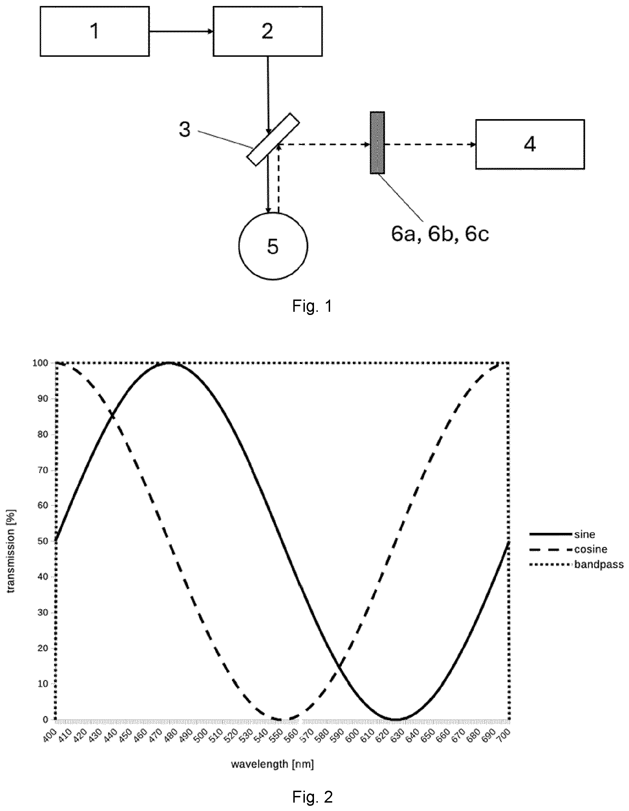

The scheme of the measuring system for performing a measurement on the eye, is depicted in Figure 1.

The arrangement of a laser 1 - a scanner 2 - a dichroic mirror 3 - a detector 4 system is commonly used in various optical imaging techniques. This setup facilitates precise control of light delivery to the sample and efficient collection of emitted signals. The laser 1 provides focused light for excitation of the fluorophores. The scanner 2 steers the laser beam across the sample. The dichroic mirror 3 directs the laser beam towards the sample while allowing the emitted fluorescence from the sample to pass through to the detector 4. In the present invention, the sample is an eye 5. In Figure 1, the excitation light is presented using a solid line, whereby the fluorescence light is presented using a dashed line.

The first component of the system is the laser 1, i.e. beam source, providing light for excitation of the fluorophores. The laser is focused onto a specific location in the tissue and scanned across the sample to sequentially produce the image. Since the (human) eye is a highly sensitive organ, lasers suitable for eye imaging must meet specific requirements to ensure safety. Therefore, the laser 1 must comply with safety standards while providing sufficient power and wavelength specificity.

Two-photon excitation requires simultaneous excitation by two photons with longer wavelength than the emitted light. The laser wavelength is critical for achieving both tissue penetration and fluorescence excitation while minimizing photodamage. Wavelength range able to excite different fluorophores is typically in the range between 700 nm to 1100 nm. Near-infrared (NIR) lasers (700-1300 nm) are commonly used in two-photon microscopy due to their deep tissue penetration, reduced scattering, and lower phototoxicity compared to visible light. Exemplary NIR lasers include titanium-sapphire lasers, which are suitable for two-photon microscopy due to their tunable wavelength range (700-1000 nm) and compatibility with multiphoton excitation of various fluorophores. Another example of suitable lasers are fiber lasers (typically operating in 1060-1300 nm range). Optical parametric oscillators may be additionally used to extend the wavelength range beyond that of Ti:Sapphire lasers, what is especially useful for red and far-red fluorophores.

Since in eye imaging techniques the exposition to laser beam should be limited to minimize photodamage and photobleaching, the preferred laser type for use in the system according to the invention is a femtosecond laser. Femtosecond lasers, for which pulse duration is typically in the femtosecond range (typically in the range of 10 fs to 200 fs) are ultrafast lasers which provide high spatial resolution and minimal heat diffusion. They allow to excite fluorophores with low photodamage and photobleaching. Ti-sapphire or fiber lasers may be femtosecond lasers. Repetition rate of such lasers is usually lower than their typical value of 80 MHz to balance fluorescence signal strength and reduce photodamage. Extending the tunability of ultrafast lasers to longer NIR wavelengths (up to 1300 nm) allows for imaging of deeper layers of the eye.

Moreover, the laser 1 should be a low power laser to allow safe imaging of sensitive structures. Additionally, the laser 1 should ensure a high-quality, stable beam for optimal resolution and contrast during imaging.

The laser 1 is placed at the rear of the optical path, behind the scanner 2 (also referred to as a scanning system), and its light is directed towards the scanner 2. The scanner's role is to direct the laser beam across the eye 5 in a controlled manner. The scanner 2 steers the laser beam across the eye 5 to form a full image in a predefined pattern, such as a line-by-line or frame-by-frame motion.

For two-photon imaging of fluorophores, specialized scanners are used, such as for example galvanometric scanners. These scanners use mirrors mounted on motors (galvanometers) to deflect the laser beam across the sample in a precise and controlled manner. The mirrors are driven by small motors, allowing rapid and precise deflection of the laser beam across the ocular tissue. Galvanometer scanners can move the laser beam rapidly to scan large areas of the retina or other eye tissues, and can achieve fine resolution, which is crucial for observing fine structures like the retinal layers. Some systems can scan in both directions, reducing image acquisition time.

Resonant scanners are a variant of galvanometer scanners that use a resonant mirror (typically a very fast oscillating mirror) to scan the laser beam over the eye. These scanners can provide fast scan rates, making them ideal for in vivo imaging of the eye. The resonant mirror oscillates at a resonant frequency (typically tens of kHz), enabling fast line scans, which is crucial for imaging living tissues where motion artifacts or changes in fluorescence may occur rapidly. These scanners can achieve scan speeds of up to 30,000 lines per second. Due to the higher scan speed, exposure to the laser is minimized, reducing photobleaching and photodamage.

Acousto-optic deflectors (AOD) are another type of scanner suitable for two-photon microscopy. They work by passing the laser through an acousto-optic medium, where the laser's path is deflected by sound waves. An acousto-optic crystal (usually made from materials like tellurium dioxide) is used to create a diffraction grating that deflects the laser beam. The deflection angle is controlled by changing the frequency of the sound waves applied to the crystal, allowing rapid beam steering. AODs are capable of fast scanning (sub-microsecond response times), which is beneficial for in vivo imaging of moving or rapidly changing ocular structures.

Another suitable type of scanner is a polygon scanner, which uses a rotating polygon mirror to scan the laser beam across the sample. The polygon mirror rotates at high speed, deflecting the laser beam at different angles to direct it to various locations on the sample (eye 5). It offers high-speed and wide-field imaging capabilities.

The scanner 2 is located in an optical path preceding a dichroic mirror 3, i.e. between the laser 1 and the dichroic mirror 3. The dichroic mirror 3, also known as a beamsplitter or optical filter, is a key optical component that directs the laser beam towards the sample (eye 5 in the present invention) while allowing the emitted fluorescence from the eye 5 to pass through to a detector 4. The dichroic mirror 3 is preferably positioned in the optical path between the laser 1 and the measured object (sample, which is the eye) at a 45-degree angle to both - the angle of incidence (AOI) for dichroic mirror 3 is thus preferably 45 degrees. In the system according to the invention, which uses fluorescence, the dichroic mirror 3 reflects the excitation wavelength and transmits the emission wavelengths. The dichroic mirror 3 thus reflects the excitation light (typically NIR) towards the measured object (the eye 5) while transmitting the longer-wavelength emitted fluorescence light to the detector 4. Therefore, the excitation light produced by the laser 1 is reflected by a dichroic mirror 3 (due to its specific wavelength-selective coating). Once the excitation light interacts with fluorescent molecules in the measured object, these molecules emit light at a different (usually longer) wavelength, which the dichroic mirror 3 is designed to transmit. Since the emission light usually has longer wavelengths, the dichroic mirror 3 is preferably a long-pass dichroic mirrors, having high reflectance at short wavelengths and high transmittance at longer wavelengths. In other words, the dichroic mirror 3 serves to direct the emitted fluorescence wavelengths from the eye 5 towards the detector 4 by transmitting the longer-wavelength emission light and reflecting shorter-wavelength excitation light.

The light from the laser 1, after being directed by the scanner 2 and reflected from the dichroic mirror 3, is focused onto the eye 5, exciting fluorophores in the eye 5. The eye 5 is positioned in the focal plane of the objective lens. The fluorophores in the eye 5 thus interact with the laser light, causing fluorescence, which is then collected by the detector 4.

The detector 4, more specifically a photodetector, is responsible for collecting the emitted signal from the fluorophores of the eye 5 after it passes through the dichroic mirror 3. This is where the fluorescence light is captured and transformed into an image or data. The signal detected by the detector 4 is then converted into an electrical signal for image processing and analysis. Due to the low signal levels and the need for high dynamic range, specialized detectors are used in two-photon microscopy.

The detector 4 may be thus for example a photomultiplier tube (PMT). PMTs operate based on the photoelectric effect. When a photon strikes a photocathode, it ejects an electron, which is then amplified through a series of dynodes (electrodes). Each dynode amplifies the signal, resulting in a measurable electrical output. PMTs can detect low levels of light, making them suitable for detecting the weak fluorescence signals in two-photon imaging. They also have a fast temporal response, allowing them to capture rapidly changing fluorescence signals in real-time imaging.

Another example of the detector 4, which is a variant of PMT, is a gallium arsenide phosphide (GaAsP) PMT. GaAsP PMTs are a specialized type of PMT with improved quantum efficiency, particularly in the visible to near-infrared range. They have greater sensitivity for detecting fluorescence signals, especially from deeper layers of the eye.

Avalanche Photodiodes (APDs), also suitable for use as the detector 4, are semiconductor-based detectors that provide high sensitivity and fast response times. When a photon strikes the semiconductor material, it generates an electron-hole pair. These charges are multiplied through an avalanche process, resulting in a detectable electrical signal. The internal multiplication process provides significant signal amplification. APDs can detect rapid changes in light intensity and have high quantum efficiency in the near-infrared region.

Another example of the detector 4 may be a hybrid detector (HyD). Hybrid detectors combine the advantages of PMTs and avalanche photodiodes (APDs), offering improved sensitivity, dynamic range, and speed. HyDs use a photocathode to convert photons into electrons, followed by an avalanche photodiode for amplification.

The detector may also be a charge-coupled devices (CCD) or complementary metal-oxide-semiconductor (CMOS) camera. These detectors capture light on a pixelated sensor, converting photons into electronic signals that are read out and processed to form an image. CCDs and CMOS cameras provide detailed spatial information about the fluorescence signal and are suitable for imaging larger areas of the eye.

High-quality objective lenses (not shown) are used for focusing the laser beam and collecting fluorescence emission. Due to broad spectrum used, these lenses need to be optimized for transmission in the visible and IR wavelength range with special coating. Great attention must also be paid to minimizing the chromatic aberrations so a high-quality focal spot can be formed. Inevitable amount of the material dispersion introduced to the pulse by transmitting the light through such objectives is typically precompensated for in dedicated optical systems, i.e. pulse shapers (e.g. based on spatial light modulators) or linear dispersion compensators (e.g. prism or grating pairs). Without such dispersion compensation the light pulses become elongated and, as a result, lack to excite the two-photon processes in the eye efficiently.

The key element of the system according to the invention is a set of spectral filters 6. Therefore, the system as described above comprises a place to insert at least one additional optical component, which is selected from a specially designed absorption filter set - a set of spectral filters 6. The set of optical spectral filters 6 serves for preparing the data ready for phasor analysis.

Each filter of the spectral filters 6 set is positioned on the optical path between the dichroic mirror 3 and the detector 4. The added spectral absorption filter selected from the set of spectral filters 6 can be placed anywhere in the detection beam path.

The set of spectral filters 6 comprises three filters: a first filter 6a which is a filter having sine-wave spectral transmission characteristics, a second filter 6b which is a filter having cosine-wave spectral transmission characteristics, and a third filter 6c which is a bandpass filter.

The first filter 6a is a filter with sine-wave spectral transmission characteristics. In the present invention, the first filter 6a filters specific fluorescence emission peaks while minimizing background noise. The first filter 6a with sine-wave spectral transmission characteristics selectively transmits or blocks light at specific wavelengths in a periodic, sinusoidal pattern. Unlike filters with a sharp cutoff or bandpass region, such filters exhibit transmission and rejection bands that follow a sinusoidal curve across the spectrum. The transmission spectrum of these filters follows a sinusoidal pattern, where transmission maxima (peaks) and minima (valleys) occur at regular wavelength intervals.

The periodicity and amplitude of the sine wave determine the filter's performance. The wavelength interval between two consecutive maxima is given by the free spectral range (FSR). FSR is typically determined by the physical properties of the filter, such as thickness, material, and refractive index. The difference between the transmission maxima and minima is given by modulation depth (the depth of the sine wave). High modulation depth leads to greater contrast and more selective wavelength transmission. Low modulation depth results in smoother transmission with less selectivity. Filters with sine-wave spectral transmission characteristics provide narrow bandwidths at the transmission peaks, allowing precise selection of specific wavelengths while rejecting others. Such filters can be designed for specific wavelength ranges. The first filter 6a is thus designed for the visible (VIS) or near-infrared (NIR) regions.

The transmission function $T(\lambda)$ of a sine-wave optical filter is typically represented as:

$$T(\lambda) = T_0 + A\sin(\frac{2\pi}{\Lambda}(\lambda - \lambda_0) + \phi)$$

where:

$T_0$ is baseline or average transmission;
A is amplitude of the sine wave, indicating the modulation depth;
$\Lambda$ is a period of the sine wave, determining the wavelength spacing between successive transmission peaks;
$\lambda_0$ is central wavelength around which the sinusoidal transmission pattern is centered;
$\phi$ is phase shift of the sine wave along the wavelength axis.

Filters with sine-wave spectral transmission characteristics are for example constructed using interference-based techniques. Therefore, the first filter 6a may be a Fabry-Pérot interferometer, composed of two parallel reflective surfaces separated by a dielectric spacer. The multiple reflections between the surfaces create constructive and destructive interference, leading to periodic transmission peaks. In another example, the first filter 6a may be a filter with dielectric multilayer coating, comprising alternating layers of materials with different refractive indices, designed to produce sinusoidal transmission characteristics through interference effects. In yet another example, the first filter 6a may be a thin-film filter, having a thin film coating deposited using precision thin-film deposition techniques to achieve the desired sinusoidal transmission profile.

The second filter 6b is a filter with cosine-wave spectral transmission characteristics. It serves for separating of wavelengths with high precision, improving the signal-to-noise ratio. The second filter 6b with cosine-wave spectral transmission characteristics is thus an optical filter that exhibits a transmission spectrum resembling a cosine-wave pattern across a specific wavelength range. Such filters are designed to provide periodic variations in transmission intensity, allowing selective wavelengths of light to pass through while attenuating others in a periodic fashion. Similarly as for sine-wave filters, the transmission spectrum of cosine-wave filters follows a sinusoidal pattern, where transmission maxima (peaks) and minima (valleys) occur at regular wavelength intervals. The transmission spectrum thus comprises periodic peaks (high transmission) and troughs (low transmission) with a cosine-like shape.

Similarly as for the first filter 6a, the periodicity and amplitude of the cosine wave determine the filter's performance. The wavelength interval between two consecutive maxima is given by the free spectral range (FSR). FSR is typically determined by the physical properties of the filter, such as thickness, material, and refractive index. The difference between the transmission maxima and minima is given by modulation depth (he depth of the cosine wave). High modulation depth leads to greater contrast and more selective wavelength transmission. Low modulation depth results in smoother transmission with less selectivity. Filters with cosine-wave spectral transmission characteristics provide narrow bandwidths at the transmission peaks, allowing precise selection of specific wavelengths while rejecting others. Such filters can be designed for specific wavelength ranges. The second filter 6b is thus designed for the visible (VIS) or near-infrared (NIR) regions.

The transmission function $T(\lambda)$ of a cosine-wave optical filter follows a cosine-wave function of wavelength, and is typically represented as:

$$T(\lambda) = T_0 + A\cos(\frac{2\pi}{\Lambda}(\lambda - \lambda_0) + \phi)$$

where:

$T_0$ is baseline or average transmission;
A is amplitude of the cosine wave, indicating the modulation depth;
$\Lambda$ is a period of the cosine wave, determining the wavelength spacing between successive transmission peaks;
$\lambda_0$ is central wavelength around which the sinusoidal transmission pattern is centered;
$\phi$ is phase shift of the cosine wave along the wavelength axis.

Filters with cosine-wave spectral transmission characteristics are typically constructed using multilayer dielectric coatings or interference filters. The multilayer structure creates constructive and destructive interference patterns, resulting in periodic transmission bands. Thus, the second filter 6b may be an interference filter, made by stacking thin dielectric layers with different refractive indices. The thickness and arrangement of these layers determine the wavelength range and periodicity of the cosine-wave transmission. To achieve more precise and complex spectral transmission patterns, the second filter 6b may be a holographic optical element (HOE) that that utilize holography principles to manipulate light waves.

In general, the specific examples of filters provided for the first filter (6a) apply to the second filter (6b), and the specific examples of filters provided for the second filter (6b) apply to the first filter (6b).

The sine- and cosine-wave spectra filters - the first filter 6a and the second filter 6b - can be made with nearly 100% modulation (transmission to extinction ratio exceeding 95%) and preferably with 300 nm FSR.

The third filter 6c is a filter with bandpass spectral transmission characteristics. In the present invention, the third filter 6c cuts off the wavelengths out of interest and normalizes the signal by measuring the total value in the band. In other words, the third filter 6c which is a bandpass filter selectively transmits light within a specific range of wavelengths (the passband) while blocking wavelengths outside this range. Such a filter is important in two-photon imaging according to the invention since it isolates the emitted fluorescence signal from background light or other unwanted signals, enabling precise detection of specific fluorophores.

The third filter's 6c performance is determined for example by the center wavelength (CWL), which is the midpoint of the bandpass filter's transmission band, typically measured in nanometers (nm). In the present invention, the third filter 6c for example has the CWL at 550 nm. The full width at half maximum (FWHM) defines the bandwidth of the bandpass filter, which is the width of the transmission band at 50% of the peak transmission. A narrow bandwidth (e.g. 10 nm) provides more selective filtering, while a broader bandwidth (e.g. 50 nm) allows more light transmission. In the present invention, the FWHM of the third filter may be for example 300 nm. Bandpass filters typically have high transmission efficiency within the passband, often exceeding 90%, to maximize the detected signal. The blocking range defines the wavelengths outside the passband that the bandpass filter is designed to block. Blocking is often expressed in terms of optical density (OD), where for example OD 4 means that $10^{-4}$ or 0.01% of light is transmitted.

The third filter 6c may be for example a hard-coated filter, made using thin-film dielectric coatings on a glass substrate, or a soft-coated filter, made using traditional deposition techniques, resulting in a multilayer coating.

The transmission range of the third filter 6c must match the emission spectrum of the fluorophore while minimizing overlap with the excitation wavelength to reduce background noise.

Preferably, the third filter 6c is a multiband bandpass filter, allowing to transmit fluorescence from multiple fluorophores simultaneously. Such filters have multiple passbands, each corresponding to a specific fluorophore.

Bandpass filters may for example isolate the fluorescence from retinal fluorophores such as green fluorescent protein (GFP) or autofluorescence of the retinal pigment epithelium (RPE). For example, a 530/30 nm filter (where 530 stands for CWL and 30 stands for FWHM) is used to detect GFP while blocking excitation light.

Figure 2 shows sine-shaped and cosine-shaped optical filter transmission characteristics (of the first filter 6a and the second filter 6b, respectively) which can be used to obtain dataset ready for phasor analysis. The output of the filters 6a, 6b is normalized and shifted to give the range of the cosine and sine functions. The bandpass filter, which is the third filter 6c, serves as a limit of out-of-band signal and a means of signal normalization. Outside the region of 400nm to 700nm the transmission is zero with the addition of bandpass third filter 6c.

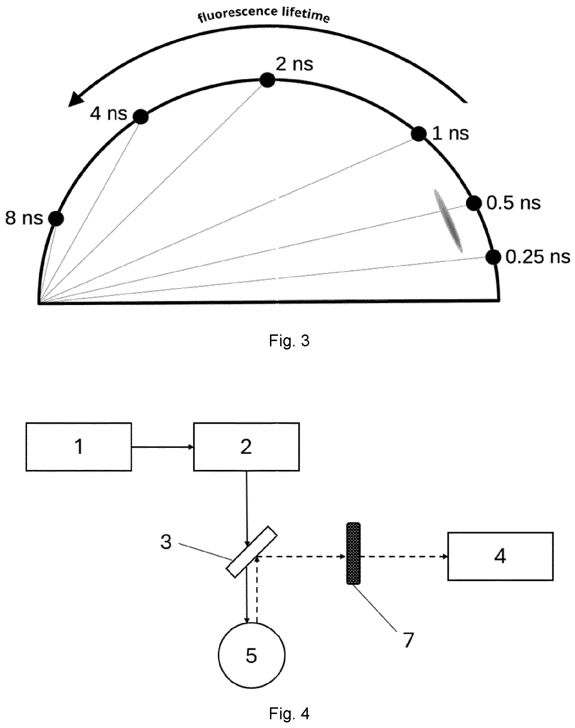

The exemplary phasor representation of the function obtained with three filters 6a, 6b, 6c is presented in Fig. 3. The semicircular arc represents the theoretical space of phasor values for decay lifetimes. Fluorescence lifetimes are mapped onto this curve, with longer lifetimes toward the left side and shorter lifetimes toward the right side of the arc. Specific fluorescence lifetimes (e.g., 8 ns, 4 ns, 2 ns, 1 ns, 0.5 ns, and 0.25 ns) are presented as black dots along the semicircle. These values demonstrate the relationship between lifetime and phasor coordinates. For mixtures, points typically fall inside the semicircle, representing a combination of multiple lifetime components. The gray shaded region (elliptically shaped) near the 0.5 ns point presents an exemplary experimental result of measurement and calculation of a fluorophore with such fluorescence decay times.

The first filter 6a and the third filter 6c may cross each other's optical path. Similarly, the second filter 6b and the third filter 6c may cross each other's optical path. In other words, they may be placed one next to another to form a stack. This may be achieved by positioning said filters together on the optical path, i.e. performing one measurement using the first filter 6a stacked with the third filter 6c, and performing another measurement using the second filter 6b stacked with the third filter 6c. Therefore, regarding the third filter 6c, in one embodiment it crosses the optical path with the first filter 6a and with the second filter 6b. In another embodiment, the third filter 6c does not cross the optical path with the first filter 6a and/or the second filter 6b, i.e. each filter 6a,6b,6c is used separately.

In the simplest embodiment of the system according to the invention, the first filter 6a, the second filter 6b and the third filter 6c are separate elements and thus are replaced in the optical path during measurements manually.

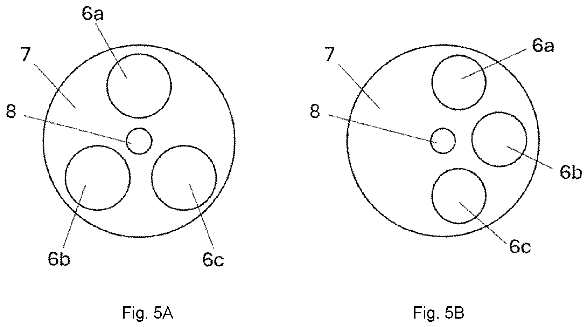

To facilitate the measurement process according to the invention, which is a series of three image acquisitions (which will be described below), the first filter 6a, the second filter 6b and the third filter 6c are be mounted in a rotatable disc 7 to quickly swap them during the procedure. This is shown in Fig. 4. The rotatable disc 7 can be rotated or turned (to a desired position). The rotatable disc 7 has an ability to be rotated manually or electrically, and during the measurement process it rotates to change the filter used. Two exemplary embodiments of the rotatable disc 7, comprising three spectral filters 6a, 6b, 6c, are shown in Fig. 5. The rotatable disc 7 may be mounted on a shaft 8. Filter position geometry in the rotatable disc 7 may be optimized depending on the optical beam size and rotation speed requirements. For example, the filters 6a, 6b, 6c may be arranged concentrically around the center (around the shaft 8) of the rotatable disc 7, as shown in Fig. 5A. In another example, the filters 6a, 6b, 6c may be positioned asymmetrically or at uneven intervals around the center of the rotatable disc 7. For example, the filters 6a, 6b, 6c may be positioned more densely on one half of the rotatable disc 7, as shown in Fig. 5B.

In one embodiment, the rotatable disc 7 may be operated manually, as mentioned above.

In another embodiment, the rotatable disc 7 may be motorized (operated electrically), what allows to swap the filters 6a, 6b, 6c quicker and without a need of any manual procedure. In such an embodiment, the shaft 8 may be for example driven by an electric motor. Arranging the set of filters 6 in the motorized rotatable disc 7 is advantageous since no external light is allowed to enter into the system, because the photomultiplier may get damaged by this light.

In yet another embodiment, the first filter 6a may be placed in the rotatable disc 7 together with the third filter 6c (as a stack of two filters), and/or the second filter 6b may be placed in the rotatable disc 7 together with the third filter 6c (as a stack of two filters). In such case, the rotatable disc 7 has two openings for filters instead of three.

Further, a method of performing measurements, more specifically a method of data acquisition, using the system as described above is provided.

In general, the method comprises performing three measurements, carried out with the successive use of one of the set of filters 6, i.e. the successive measurements using each of three proposed spectral filters 6a, 6b, 6c. This enables the performance of a computational phasor spectral analysis and, in effect, allows for identification of substances emitting the measured fluorescence light from a given location.

The data acquisition procedure is as follows.

Firstly, the excitation light from the laser 1 is directed towards the object to be measured (the eye 5). The excitation light is reflected by the dichroic mirror 3 and the emission light, emitted by the fluorophores in the measured object, is transmitted by the dichroic mirror 3 towards the detector 4.

Capturing of the emission light from the measured object by the detector 4 is performed in a frame series comprising three sequences. Thus, the measurement of a frame series is performed. One series may comprise, for example, 30 frames (with 10 frames for each sequence). However, the number of frames in one series may be different. In the simplest embodiment, even one frame may be used, although this may eventually result in poor spatial resolution.

In the first sequence, the emission light (from the measured object) is passed through the first filter 6a having sine-wave spectral transmission characteristics. In the second sequence, the emission light is passed through a second filter 6b having cosine-wave spectral transmission characteristics. In the third sequence, the emission light is passed through a third filter 6c being a bandpass filter. The transmission range of the third filter 6c matches the emission spectrum of the fluorophore of interest. In an obvious manner, each filter 6a,6b,6c is positioned on the optical path between the dichroic mirror 3 and the detector 4, as described with reference to the system.

A series of frames can be computationally corrected for motion in postprocessing by aligning single frames to match each other in the spatial position to compensate for shifts, improving spatial resolution and accuracy. A frame series can be repeated to further enhance the image contrast by adding more images of the same retinal spot to the data set. For example, two or three series, each with at least one frame (for example 30 frames) may be performed.

In each frame series the measurement is performed using each of the three filters 6a, 6b, 6c, preferably with even distribution of frames per each filter 6a, 6b, 6c. For example, first measurement (first sequence) may be performed using the first filter 6a, second measurement (second sequence) may be performed using the second filter 6b, and third measurement (third sequence) may be performed using the third filter 6c, where each measurement preferably comprises capturing plurality of frames. However, the order of measurements using different filters may be any other, for example in the first sequence the first filter 6a may be used, followed by the second sequence using the third filter 6c, followed by the third sequence using the second filter 6b. In another example, the second filter 6b may be followed by the third filter 6c, which may be followed by the first filter 6a, etc.

For example, in the case of 30-frame series, 10 frames are captured with the first filter 6a which is a sine-wave filter, 10 frames are captured with the second filter 6b which is a cosine-wave filter and 10 frames are captured with the third filter 6b which is a bandpass filter. Such a series may be then repeated to further enhance the image contrast.

In one embodiment, during each series, the filters 6a, 6b, 6c are changed manually. In such case the time of each series is longer because one filter has to be removed and the consecutive filter has to be mounted in the optical path of the system.

In another example, the filters 6a, 6b, 6c are mounted in a rotatable disc 7. This arrangement is shown in Fig. 4.

In one embodiment, the rotatable disc 7 may be is operated manually. In such case the measurement time for each series is shorter than in the case of separate filters (as in previous embodiment), since the only manual operation performed is rotating the disc which is already mounted in the system.

To maximize the speed of the measurement, the filters 6a, 6b, 6c are mounted in a rotatable disc 7 which is for example operated electrically (motorized). This allows for automatic change of the filter during each series.

In yet another embodiment of the method, one sequence may be performed using both the first filter 6a and the third filter 6c, and another sequence may be performed using both the second filter 6b and the third filter 6c. In such case, one frame series comprises two sequences instead of three.

For example, imaging the retina of the eye 5 may be performed over a 3-second window with 30 frames captured at 10 frames per second. The frames in one series are captured using each of the first filter 6a, the second filter 6b and the third filter 6c, while each filter 6a,6b,6c captures 1/3 of the overall number of the frames.

In the next step, which is performed after data acquisition, the frame series are integrated or averaged in postprocessing to reduce noise or highlight specific temporal dynamics.

Short series of data recorded with the complete set of spectral filters 6 minimize the risk of the patient's eye moving during one series.

The data obtained using the set of optical spectral filters 6 is data suitable for performing phasor analysis, which is an analysis method known in the art. The exemplary phasor representation of the function obtained with three filters 6a, 6b, 6c is presented in Fig. 3, as described above.

The embodiments allow to perform spectroscopic analysis of the imaged retinal fluorophores without engaging fluorescence lifetime analysis or direct spectral measurements. Moreover, it allows for performing a spectroscopic analysis of the imaged retinal fluorophores in a short time, applicable to in-vivo measurements, avoiding image artefacts due to the patient's eye movement. Furthermore, it allows for performing a spectroscopic analysis of the imaged retinal fluorophores in the low signal level regime, applicable to in-vivo measurements, avoiding dangerous excess light intensity.

LIST OF REFERENCE NUMERALS USED

| | |
|---|---|
| 1 | laser |
| 2 | scanner |
| 3 | dichroic mirror |
| 4 | detector |
| 5 | eye |
| 6 | set of spectral filters |
| 6a | first filter |
| 6b | second filter |
| 6c | third filter |
| 7 | rotatable disc |
| 8 | shaft |

**Claims**

1. A two-photon fluorescence lifetime imaging system for imaging fluorophores in a living eye, comprising a laser (1) for providing excitation light; a scanner (2) for directing the laser (1) light across the measured object; a detector (4) for collecting the emitted light from the fluorophores; a dichroic mirror (3) positioned in the optical path between the laser (1) and the measured object, whereby the dichroic mirror (3) reflects the excitation light and transmits the emission light from the measured object towards the detector (4), a set of spectral filters (6) positioned on the optical path between the dichroic mirror (3) and the detector (4), **characterized in that** the set of spectral filters (6) comprises:

   a first filter (6a) having sine-wave spectral transmission characteristics,
   a second filter (6b) having cosine-wave spectral transmission characteristics, and
   a third filter (6c) being a bandpass filter, whereby the transmission range of the third filter (6c) matches the emission spectrum of the fluorophore of interest.

2. The system according to claim 1, wherein the first filter (6a) and/or the second filter (6b) is a Fabry-Pérot interferometer or a filter with dielectric multilayer coating or a thin-film filter or a holographic optical element.

3. The system according to claim 1 or 2, wherein the free spectral range of the first filter (6a) and/or the second filter (6b) is 300 nm.

4. The system according to claim 1 or 2 or 3, wherein the first filter (6a), the second filter (6b) and the third filter (6c) are arranged separately.

5. The system according to any one of the claims from 1 to 3, wherein the first filter (6a), the second filter (6b) and the third filter (6c) are mounted in a rotatable disc (7).

6. The system according to claim 5, wherein the rotatable disc (7) is electrically operable.

7. The system according to claim 5 or 6, wherein the first filter (6a), the second filter (6b) and the third filter (6c) are arranged concentrically around the center of the rotatable disc (7).

8. The system according to claim 5 or 6, wherein the first filter (6a), the second filter (6b) and the third filter (6c) are arranged asymmetrically around the center of the rotatable disc (7) or at uneven intervals around the center of the rotatable disc (7).

9. The system according to any of the preceding claims, wherein the system further comprises a number of objective lenses for focusing the laser (1) light and collecting fluorescence emission light.

10. The system according to any of the preceding claims, wherein the laser (1) emits light in the wavelength range between 700 nm to 1100 nm.

11. The system according to any of the preceding claims, wherein the third filter (6c) is a multiband bandpass filter.

12. The system according to any of the preceding claims, wherein the third filter (6c) has a center wavelength in the range from 400 to 700 nm.

13. A method for two-photon fluorescence lifetime imaging of fluorophores in a living eye performed in the system according to any of the claims from 1 to 12, comprising:

a) sending excitation light to an object to be measured;
b) reflecting the excitation light and transmitting emission light from the measured object towards an detector (4), whereby capturing the emission light from the measured object by the detector (4) is performed in at least one frame series comprising sequences captured using a set of three spectral filters (6), each sequence having at least one frame, whereby said frame series comprises capturing:

i. a sequence in which the emission light passes through a first filter (6a) having sine-wave spectral transmission characteristics,
ii. a sequence in which the emission light passes through a second filter (6b) having cosine-wave spectral transmission characteristics,
iii. a sequence in which the emission light passes through a third filter (6c) being a bandpass filter, the transmission range of the third filter (6c) matching the emission spectrum of the fluorophore of interest,

whereby the steps i, ii, iii are performed in any order.

14. The method according to claim 13, wherein the steps i and iii are performed simultaneously and/or the sequences ii. and iii. are performed simultaneously.

15. The method according to claim 13 or 14, wherein the method further comprises a step of performing spectral phasor analysis of the data obtained according to steps i, ii, iii.

**Patentansprüche**

1. Zwei-Photonen-Fluoreszenzlebensdauer-Bildgebungssystem zum Abbilden von Fluorophoren in einem lebenden Auge, umfassend einen Laser (1) zum Bereitstellen von Anregungslicht; einen Scanner (2) zum Richten des Lichts des Lasers (1) über das Messobjekt; einen Detektor (4) zum Sammeln des von den Fluorophoren emittierten Lichts; einen dichroitischen Spiegel (3), der im Strahlengang zwischen dem Laser (1) und dem Messobjekt positioniert ist, wobei der dichroitische Spiegel (3) das Anregungslicht reflektiert und das Emissionslicht vom Messobjekt zum Detektor (4) durchlässt, einen Satz von Spektralfiltern (6), die im Strahlengang zwischen dem dichroitischen Spiegel (3) und dem Detektor (4) positioniert sind, **dadurch gekennzeichnet, dass** der Satz von Spektralfiltern (6) Folgendes umfasst: einen ersten Filter (6a) mit sinusförmigen spektralen Transmissionseigenschaften, einen zweiten Filter (6b) mit kosinusförmigen spektralen Transmissionseigenschaften und einen dritten Filter (6c), der ein Bandpassfilter ist, wobei der Transmissionsbereich des dritten Filters (6c) dem Emissionsspektrum des betreffenden Fluorophors entspricht.

2. System nach Anspruch 1, wobei der erste Filter (6a) und/oder der zweite Filter (6b) ein Fabry-Perot-Interferometer oder ein Filter mit dielektrischer Mehrschichtbeschichtung oder ein Dünnschichtfilter oder ein holografisches optisches Element ist.

3. System nach Anspruch 1 oder 2, wobei der freie Spektralbereich des ersten Filters (6a) und/oder des zweiten Filters (6b) 300 nm beträgt.

4. System nach Anspruch 1 oder 2 oder 3, wobei der erste Filter (6a), der zweite Filter (6b) und der dritte Filter (6c) separat angeordnet sind.

5. System nach einem der Ansprüche 1 bis 3, wobei der erste Filter (6a), der zweite Filter (6b) und der dritte Filter (6c) in einer drehbaren Scheibe (7) angebracht sind.

6. System nach Anspruch 5, wobei die drehbare Scheibe (7) elektrisch betätigbar ist.

7. System nach Anspruch 5 oder 6, wobei der erste Filter (6a), der zweite Filter (6b) und der dritte Filter (6c) konzentrisch um den Mittelpunkt der drehbaren Scheibe (7) angeordnet sind.

8. System nach Anspruch 5 oder 6, wobei der erste Filter (6a), der zweite Filter (6b) und der dritte Filter (6c) asymmetrisch um den Mittelpunkt der drehbaren Scheibe (7) oder in ungleichmäßigen Abständen um den Mittelpunkt der drehbaren Scheibe (7) angeordnet sind.

9. System nach einem der vorhergehenden Ansprüche, wobei das System ferner eine Anzahl von Objektivlinsen zum Fokussieren des Lichts des Lasers (1) und zum Sammeln von Fluoreszenzemissionslicht umfasst.

10. System nach einem der vorhergehenden Ansprüche, wobei der Laser (1) Licht im Wellenlängenbereich zwischen 700 nm und 1100 nm emittiert.

11. System nach einem der vorhergehenden Ansprüche, wobei der dritte Filter (6c) ein Multiband-Bandpassfilter ist.

12. System nach einem der vorhergehenden Ansprüche, wobei der dritte Filter (6c) eine Mittenwellenlänge im Bereich von 400 bis 700 nm aufweist.

13. Verfahren zur Zwei-Photonen-Fluoreszenzlebensdauer-Bildgebung von Fluorophoren in einem lebenden Auge, das in dem System nach einem der Ansprüche 1 bis 12 durchgeführt wird, umfassend:

    a) Senden von Anregungslicht an ein zu messendes Objekt;
    b) Reflektieren des Anregungslichts und Durchlassen von Emissionslicht von dem Messobjekt zu einem Detektor (4), wobei das Erfassen des Emissionslichts von dem Messobjekt durch den Detektor (4) in mindestens einer Einzelbildserie erfolgt, die Sequenzen umfasst, die unter Verwendung eines Satzes von drei Spektralfiltern (6) erfasst werden, wobei jede Sequenz mindestens ein Einzelbild aufweist, wobei die Einzelbildserie das Erfassen von Folgendem umfasst:

       i. eine Sequenz, in der das Emissionslicht durch einen ersten Filter (6a) mit sinusförmigen spektralen Transmissionseigenschaften hindurchtritt,

       ii. eine Sequenz, in der das Emissionslicht durch einen zweiten Filter (6b) mit kosinusförmigen spektralen Transmissionseigenschaften hindurchtritt,
       iii. eine Sequenz, in der das Emissionslicht durch einen dritten Filter (6c) hindurchtritt, der ein Bandpassfilter ist, wobei der Transmissionsbereich des dritten Filters (6c) dem Emissionsspektrum des betreffenden Fluorophors entspricht,

    wobei die Schritte i, ii, iii in beliebiger Reihenfolge durchgeführt werden.

14. Verfahren nach Anspruch 13, wobei die Schritte i und iii gleichzeitig durchgeführt werden und/oder die Sequenzen ii. und iii. gleichzeitig durchgeführt werden.

15. Verfahren nach Anspruch 13 oder 14, wobei das Verfahren ferner einen Schritt des Durchführens einer spektralen Phasoranalyse der gemäß den Schritten i, ii, iii erhaltenen Daten umfasst.

**Revendications**

1. Un système d'imagerie par durée de vie de fluorescence à deux photons pour l'imagerie de fluorophores dans un œil vivant, comprenant un laser (1) pour fournir une lumière d'excitation ; un scanner (2) pour diriger la lumière laser (1) à travers l'objet à mesurer ; un détecteur (4) pour collecter la lumière émise par les fluorophores ; un miroir dichroïque (3) placé dans le trajet optique entre le laser (1) et l'objet mesuré, le miroir dichroïque (3) réfléchissant la lumière d'excitation et transmettant la lumière d'émission provenant de l'objet mesuré vers le détecteur (4), un ensemble de filtres spectraux (6) disposés sur le trajet optique entre le miroir dichroïque (3) et le détecteur (4), **caractérisé en ce que** l'ensemble de filtres spectraux (6) comprend : un premier filtre (6a) présentant des caractéristiques de transmission spectrale sinusoïdales, un deuxième filtre (6b) présentant des caractéristiques de transmission spectrale cosinusoïdales, et un troisième filtre (6c) qui est un filtre passe-bande, la plage de transmission de ce troisième filtre (6c) correspondant au spectre d'émission du fluorophore concerné.

2. Le système selon la revendication 1, dans lequel le premier filtre (6a) et/ou le deuxième filtre (6b) est un interféromètre de Fabry-Pérot, ou un filtre à revêtement multicouche diélectrique, ou un filtre à couche mince, ou un élément optique holographique.

3. Le système selon la revendication 1 ou 2, dans lequel la plage spectrale libre du premier filtre (6a) et/ou du deuxième filtre (6b) est de 300 nm.

**4.** Le système selon la revendication 1, 2 ou 3, dans lequel le premier filtre (6a), le deuxième filtre (6b) et le troisième filtre (6c) sont disposés séparément.

**5.** Le système selon l'une quelconque des revendications 1 à 3, dans lequel le premier filtre (6a), le deuxième filtre (6b) et le troisième filtre (6c) sont montés sur un disque rotatif (7).

**6.** Le système selon la revendication 5, dans lequel le disque rotatif (7) est actionnable électriquement.

**7.** Le système selon la revendication 5 ou 6, dans lequel le premier filtre (6a), le deuxième filtre (6b) et le troisième filtre (6c) sont disposés de manière concentrique autour du centre du disque rotatif (7).

**8.** Le système selon la revendication 5 ou 6, dans lequel le premier filtre (6a), le deuxième filtre (6b) et le troisième filtre (6c) sont disposés de manière asymétrique autour du centre du disque rotatif (7) ou à intervalles irréguliers autour du centre du disque rotatif (7).

**9.** Le système selon l'une quelconque des revendications précédentes, dans lequel le système comprend en outre un certain nombre de lentilles d'objectif pour focaliser la lumière laser (1) et collecter la lumière d'émission de fluorescence.

**10.** Le système selon l'une quelconque des revendications précédentes, dans lequel le laser (1) émet de la lumière dans la plage de longueurs d'onde entre 700 nm et 1100 nm.

**11.** Le système selon l'une quelconque des revendications précédentes, dans lequel le troisième filtre (6c) est un filtre passe-bande multibande.

**12.** Le système selon l'une quelconque des revendications précédentes, dans lequel le troisième filtre (6c) a une longueur d'onde centrale dans la plage entre 400 et 700 nm.

**13.** Un procédé d'imagerie par durée de vie de fluorescence à deux photons de fluorophores dans un œil vivant, mis en œuvre dans le système selon l'une quelconque des revendications 1 à 12, comprenant :

a) émission d'une lumière d'excitation vers un objet à mesurer ;
b) réflexion de la lumière d'excitation et transmission de la lumière d'émission provenant de l'objet mesuré vers un détecteur (4), la capture de la lumière d'émission provenant de l'objet mesuré par le détecteur (4) étant effectuée dans au moins une série d'images comprenant des séquences capturées à l'aide d'un ensemble de trois filtres spectraux (6), chaque séquence comportant au moins une image, ladite série d'images comprenant la capture :

i. d'une séquence dans laquelle la lumière d'émission traverse un premier filtre (6a) présentant des caractéristiques de transmission spectrale sinusoïdales,
ii. d'une séquence dans laquelle la lumière d'émission traverse un deuxième filtre (6b) présentant des caractéristiques de transmission spectrale cosinusoïdales,
iii. d'une séquence dans laquelle la lumière d'émission traverse un troisième filtre (6c) qui est un filtre passe-bande, la plage de transmission du troisième filtre (6c) correspondant au spectre d'émission du fluorophore concerné,

les étapes i, ii et iii étant exécutées dans n'importe quel ordre.

**14.** Le procédé selon la revendication 13, dans lequel les étapes i et iii sont exécutées simultanément et/ou les séquences ii. et iii. sont exécutées simultanément.

**15.** Le procédé selon la revendication 13 ou 14, dans lequel le procédé comprend en outre une étape consistant à effectuer une analyse spectrale par phaseurs des données obtenues selon les étapes i, ii et iii.

Fig. 1

Fig. 2

Fig. 3

Fig. 4

Fig. 5A

Fig. 5B

**EP 4 570 163 B1**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2022204248 A1